# EUROPEAN PATENT SPECIFICATION

(11) **EP 1 416 054 B1**
(45) Date of publication and mention of the grant of the patent: **17.08.2005**
(21) Application number: 02380224.2
(22) Date of filing: 31.10.2002
(51) Int. Cl.: C12P 35/04, C12N 9/84, C07D 501/36

(54) **Simple enzymatic process for preparing cefazolin**
Einfaches enzymatisches Verfahren zur Herstellung von Cefazolin
Procédé enzymatique simple pour préparer du cefazolin

(43) Date of publication of application: 06.05.2004
(73) Proprietor: Bioferma Murcia, S.A., 30840 Alhama de Murcia, Murcia (ES)
(72) Inventor: Sanchez Ferrer, Alvaro, 30820 Alcantarilla (Murcia) (ES); Garcia Carmona, Francisco, 30500 La Alcaina - Molina De Segura (ES)
(74) Representative: Davila Baz, Angel

(56) References cited:
- WO-A-02/086143
- US-A- 5 677 141
- US-B1- 6 465 227
- FERNANDEZ-LAFUENTE R ET AL: "Chemoenzymatic One-pot Synthesis of Cefazolin from Cephalosporin C in Fully Aqueous Medium, Involving Three Consecutive Biotransformations Catalyzed by D-Aminoacid Oxidase, Glutaryl Acylase and Penicillin G Acylase" TETRAHEDRON LETTERS, ELSEVIER SCIENCE PUBLISHERS, AMSTERDAM, NL, vol. 38, no. 26, 30 June 1997 (1997-06-30), pages 4693-4696, XP004074870 ISSN: 0040-4039
- WON J I ET AL: "THE EFFECT OF 2-MERCAPTO-5-METHYL-1,3,4-THIADIAZOLE ON ENZYMATIC SYNTHESIS OF CEFAZOLIN" APPLIED BIOCHEMISTRY AND BIOTECHNOLOGY, CLIFTON, NJ, US, vol. 69, no. 1, January 1998 (1998-01), pages 1-9, XP001105929 ISSN: 0273-2289
- JUSTIZ O H ET AL: "One-pot chemoenzymatic synthesis of 3'-funcionalized cephalosporines (cefazolin) by three consecutive biotransformations in ully aqueous medium" JOURNAL OF ORGANIC CHEMISTRY, AMERICAN CHEMICAL SOCIETY. EASTON, US, vol. 62, no. 26, 1997, pages 9099-9106, XP002177920 ISSN: 0022-3263
- PARK C B ET AL: "PENICILLIN ACYLASE-CATALYZED SYNTHESIS OF CEFAZOLIN IN WATER-SOLVENT MIXTURES: ENHANCEMENT EFFECT OF ETHYL ACETATE AND CARBON TETRACHLORIDE ON THE SYNTHETIC YIELD" JOURNAL OF MOLECULAR CATALYSIS. B, ENZYMATIC, ELSEVIER, AMSTERDAM,, NL, vol. 9, no. 4-6, 21 April 2000 (2000-04-21), pages 275-281, XP000989461 ISSN: 1381-1177
- "The Merck Index on CD-ROM, Version 12:2" MERCK INDEX ON CD-ROM, XX, XX, 1997, XP002177921

## Description

### Introduction

The invention relates to a process for preparing β-lactam antibiotics. In particular, the invention relates to an enzymatic process for the preparation of 7-(1H-tetrazol-1-yl) acetamido-(2-methyl-1,3,4-thiadiazol-5-yl)-thiomethyl-3-cephem 4-carboxylic acid, also known as cefazolin.

Cefazolin is a second generation semi-synthetic cephalosporin obtained by chemical modification of the β-lactam nucleus of cephalosporin C at 7- and 3-position to give the following chemical structure (I):

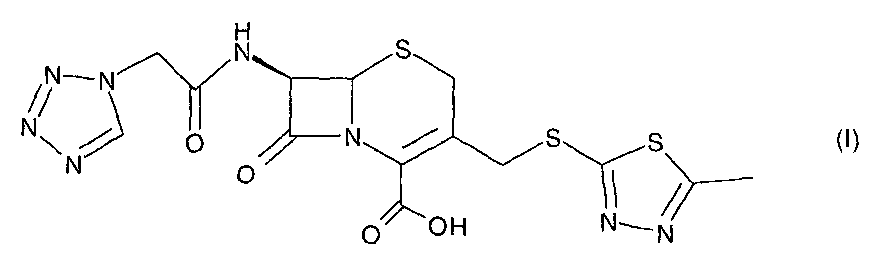

The process to produce cefazolin was first disclosed and claimed in US 3,516,997 (Fujisawa), and involves reacting the corresponding 7-aminocephalosporin with an appropriate acid or reactive derivates thereof to achieve acylation of the amino function in the 7-position of the cephalosporin. This acylation is carried out in the presence of an inert solvent, such as acetone, dioxane, acetonitrile, chloroform, methylene chloride, ethylene chloride, tetrahydrofuran or pyridine, and the like, or in a mixture of such solvents with water or with any other suitable dissolvent. For this process it is necessary to isolate the 7- amino cephalosporin derivate named 7-amino-3-(2-methyl-1,3,4-thiadiazol-5-yl) thiomethyl-3-cephem-4-carboxylic acid, hereinafter indicated as TDA. The overall yield of this process is low, as described in GB 1,565,053.

Alternative methods have been used for accomplishing acylation of 7-amino cephalosporanic acid (7-ACA) or derivates of 7-ACA in which the acetoxy function in the 3-position has been displaced. US 3,502,665 describes the acylation of 7-ACA or an acid addition salt thereof by treatment with an acyl halide under substantially anhydrous conditions and in an inert Lewis base liquid of the N,N-dialkylamide type. Among later bases, US 3,954,745 uses to carry out the reaction. 1H-tetrazol-1-acetyl chloride in N,N-dimethylacetamide and coupling the acid chloride with the DMF-solvate of the hydrochloride salt of 7-TDA to yield cefazolin. However, the yields are low and also the acylating agent, the tetrazoleacetyl chloride, because of its inherent instability, is difficult to isolate and store at room temperature. Another limitation of this process is that acylation is to be carried out in strictly anhydrous conditions as described in US 5,739,346.

US 5,945,532 describes the production of reactive derivates of 1H-tetrazol-1-acetic acid prepared by reacting it with N,N-dimethyl-formaminiun chloride chlorosulphate (DFCCS) in dichloromethane at -20°C. The final acylation reaction is carried out between the acylating agent and TDA at a temperature ranging from -70°C to -50°C with low yields of cefazolin of about from 53 to 61%.

As an alternative to the acid chloride, a mixed anhydride can be used for acylation, as described in EP 0 846 695. It uses pivaloyl chloride, strict anhydrous conditions at temperatures below zero.

The above described acylation reactions never come to completion and, at the point when the work up is started, the reaction mixture will contain the desired products as well as unreacted starting materials and possible byproducts. After the acylation, the protective groups, if any, have to be removed either *in situ* or after the protected intermediate product has been isolated. The working up of the intermediate product and the removal of the protective groups generally involves at least one step in which the product is contacted with water or an aqueous solvent. During the deprotection operation great care must be taken to avoid cleavage of the acyl bond just established. In addition, co-precipitation of the impurities contained in the crude products, which are not very much different from the similar acid-base properties of the β-lactam desired product, may easily take place when the crude product is re-precipitated or recrystallized and it can therefore be difficult to achieve a high purity of the product and a high yield at the same time. Alternatives to remove these impurities in crude products are described in US 3,979,383 by using an anion exchange resin such as Amberlite IRA-458 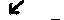 or by using sodium sulfite or sodium dithionite to decolorize impurities, as described in US 4,115,645.

As an alternative to the chemical acylating methods outlined above, the enzymatic acylation of an amino β-lactam with an acylating agent may be performed in the presence of a suitable amidase or acylase whereby the desired β-lactam is formed. For many years now, penicillin G amidase or acylase (EC. 3.5.1.11), hereinafter indicated as PGA, have been attempted to be used as a catalyst for this synthetic reaction (C.A. Claridge *et al.,* Nature, vol. 187, 237, 1960; M. Cole, Biochem. J., vol. 115, 757, 1969).
amide or a lower alkyl, polyethylenglycol or ethylenglycol ester. Most of these procedures use activated side chains derivatives such as D-(-)-phenylglycine amide, D-(-)-phenylglycine methyl ester, D-(-)-4-hydroxyphenylglycine methyl ester, D-(-)-2,5-dihydro-phenylglycine amide, D-(-)-2,5-dihydro-phenylglycine methyl ester, and amino β-lactams such as 6-amino-penicillanic acid (6-APA), 7-amino-3-chloro-3-cephem-4-carboxylic acid (7-ACCA), 7-aminodesacetoxy cephalosporonic acid (7-ADCA) and 7-amino-3-[(Z)-1-propenyl]-3-4-carboxylic acid (7-PACA). With combination of the above side chains and the amino β-lactam intermediate semisynthetic antibiotics such as amoxicillin (WO 97/04086; US 5,753,458; US 5,922,907; WO 96/02663; US 6,1565,334), ampicillin (WO 92/01061; EP 0 473 008; US 5,525,483; WO 97/04086; WO 98/56945; WO 98/56946; WO 99/15531; WO 99/15532; DE 19 823 332; WO 99/20786), cefadroxil (EP 0 473 008; WO 93/12250; US 5,922,907; US 6,156,534; WO 99/20786), cephalexin (WO 92/01061; EP 0 473 008; WO 93/12250; WO 96/23897; EP 0 730 035; EP 0 730 036; WO 97/04086; WO 98/56944; WO 99/20786), cefaclor (EP 0 473 008; EP 0 730 035; EP 0 730 036; WO 97/04086; WO 99/20786) and cefprozil (US 5,922,907; US 6,156,534) has been synthesized.

A few enzymatic work, however, has been carried out on 7-aminocephalosporanic acid (7-ACA) as an amino β-lactam nucleus. The synthesis of cephalothin has only been described only in two patents (US 4,340,672, ES 2 036 149) and just a short mention in a table is made for the 3-thiomethyl derivative of 7-ACA, i.e. TDA, used in the synthesis of cefazolin (JP 56,045,196) with a cell-free extract containing PGA. Apart from the latter patent, only few scientific papers have been published for the enzymatic synthesis of cefazolin with no optimized conditions (R. Muneyuki *et al.,* Chem. Ind., vol. 7, 159, 1981; M. Kostadinov *et al.,* Appl. Biochem. Biotechnol., vol 33, 177, 1992; R. Fernandez-La fuente *et al.,* Tetrahedron Lett., vol 38, 4693, 1997; O.H. Jústiz *et al.,* J. Org. Chem., vol 62, 9099, 1997; J.I. Won *et al.,* Appl. Biochem. Biotechnol., vol 69, 1, 1998).

In addition to the above mentioned patents, there are now numerous patent applications which have already been published, as well as granted patents, but the industrial use of the enzymatic synthesis of β-lactam antibiotics using PGA is quite limited since a high conversion (about 95-98 %) of β-lactam antibiotic per amount of β-lactam nucleus used has to be obtained.

Acceptable yields can be obtained by applying a high molecular ratio between the acylating agent and the β-lactam nucleus, as it is well known from literature, for example WO 92/01061 and EP 0 730 036. However, this approach introduces several problems under the economic point of view:
a) the concomitant unacceptable cost increase of the pure activated acylating agent and the need of recycling its free acid form after the enzymatic reaction.
b) the presence of by-products which are difficult or impossible to eliminate from the desired final penicillins or cephalosporins at the end of the reaction, which hampers the downstream processing, resulting in uneconomical losses of the product.
c) the inherent solubility problems of the substrates and products, due to the high concentrations needed, which leads to severe mass-transfer problems and also their precipitation in the enzyme carrier macropores. The latter effect reduces catalytic efficiency and the number of reuses of the enzyme.
d) the high presence of acids or bases consumed or produced during the catalysis due to the coupling reaction together with the diffusion of the substrates and products give rise to pH gradients within the enzyme carrier. These gradients cause sub-optimal reaction rates, reduced conversion and a decrease in enzyme stability.

Another important economical aspect apart from high yields to reduce costs is the possibility of using moist crude β-lactam nucleus isolated from the aqueous solutions as a starting point in the economic process, as mentioned in EP 0 730 036. However, this is hampered by the presence of traces of penicillin G or cephalosporin C side chains which inhibit PGA, reducing conversion as described in EP 0 730 036.

The above-mentioned problems are more dramatic in cefazolin, where only 7-ACA can be used as amino β-lactam. The 7-ACA is firstly enzymatically acylated at 7 position with a tetrazolyl acetic acid (TzAA) derivative, such as its corresponding methyl ester (TzAAMe) and then, it is chemically modified at the 3-position at a temperature between 60 -and 70°C with 2-mercapto-5-methyl-1,3,4-thiadiazole (MMTD) to give cefazolin, following the scheme:

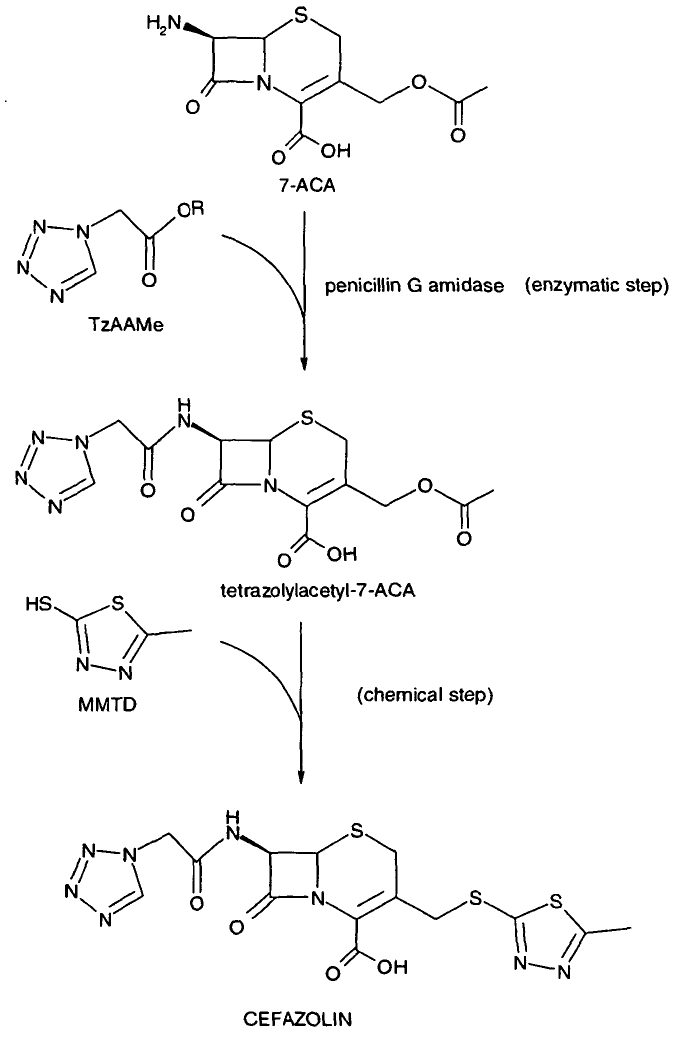

This route has the disadvantage of the excess amount of MMTD used in the second chemical reaction to enhance cefazolin yield, co-precipitates with cefazolin crystals as impurity, giving rise to a product with low purity and dark brown color, (a) further step(s) of purification being necessary, e.g. by ion exchange chromatography. The latter purification reduces the cefazolin yield to a non-commercial level.

The alternative of TDA as a substrate of PGA for the enzymatic synthesis of cefazolin has been ruled out due to the very low solubility and the poorer affinity of TDA toward the PGA active site (O.H. Jústiz *et al.,* J. Org. Chem., vol 62, 9099, 1997) In addition, the traces of MMTD usually present in commercial TDA, poisons PGA (J. I. Won *et al,* App. Biochem. Biothecnol., vol 69, 1, 1998), giving rise to a low yield of enzymatic conversion of TDA into cefazolin, and a low number of reuses of PGA. Both factors are of critical economic relevance for developing an enzymatic industrial process.

The method for the preparation of TDA (or TDC), starting with the chemical reaction between cephalosporin C and MMTD, followed by the deacetylation of the 7-position using D-amino acid oxidase followed by glutaryl-7ACA acylase, is well-known (see US 6465227B1 and US 5677141).

To solve the above mentioned problems related to the poisoning and coloring effect of MMTD, a new chemical-enzymatic-enzymatic has been described (European Patent Application 01201426.2, European Patent Application 01201699.4) in which cephalosporin C [3-acetoxymethyl-7-(D-5-amino-5-carboxypentanamido)-3-cephem-4-carboxylic acid] is converted into its corresponding 3-thiomethyl derivate [7-(D-5-amino-5-carboxypentanamido)-3-(5-methyl-1,3,4-thiadiazole-2-yl)thiomethyl-3-cephem- 4-carboxylic acid], hereinafter indicated as TDC. After chemical reaction the excess of MMTD is first removed by precipitation at acidic pH, and then, the remaining traces and color are removed by passing through an anion exchange column Amberlite IRA-400® (Röhn and Haas). This clean TDC is then enzymatically converted into TDA by two consecutive reactions with D-amino acid oxidase and glutaryl-7-ACA acylase (European Patent Application 01201699.4). The resulting high purity (about 98-99 %) TDA is almost free of MMTD, which avoids any poisoning of PGA. This procedure is known commercially as 'the TD3 process'.

The object of the present invention is to provide for the first time a simple (batch mode) and economically feasible method to produce cefazolin enzymatically by using TDA, as substrate with a high conversion and with no contamination of trace amounts of TDA or MMTD.

### Summary of the invention

According to the invention, a process for preparing the 7-(1-H-tetrazol-1-yl) acetamide-3-(2-methyl-1,3,4-thiadiazol-5-yl)thiomethyl-3-cephem-4-carboxylic acid or cefazolin of the formula: 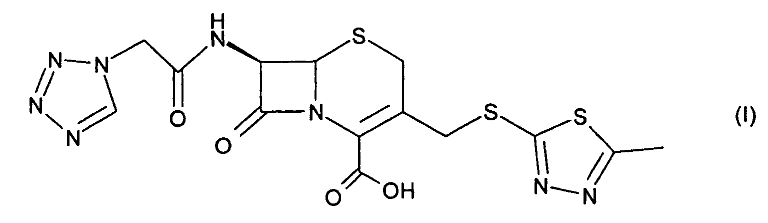 is provided, comprising catalysing the acylation of the amino β-lactam, the 7-amino-3-[5methyl-1,3,4-thiadiazol-2-yl)thiomethyl]-3-cephem-4-carboxylic acid, of the formula: 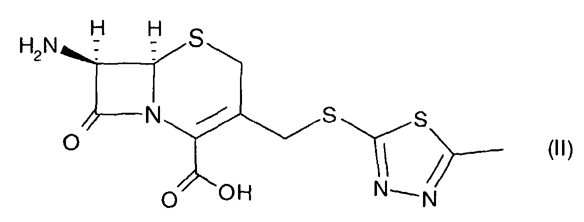 with an activated crude acylating agent derived from acetic acid of the formula: 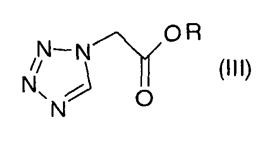 wherein R is an ester or amide of said acid, in the presence of a biocatalyst comprising penicillin amidase (EC 3.5.1.11) with a conversion of compound II into compound I above 97%, precipitating the desired β-lactam antibiotic by adding an inorganic acid to lower the pH below 3.5 at temperature in the range of 0°C to +10°C without the precipitation of remaining compound II in the acidic range of pH, and with an overall molar yield of compound I above 88% with a purity above 99%.

It has now, surprisingly, been found that the solubility of compound II (TDA) may very efficiently be controlled by adding an inorganic base, independently if the TDA comes from a TDA solution, or a moist cake of the TDA after crystallization without drying or from solids dry crystals. Then, by the process of this invention, it is possible and economically attractive to use a crude solution of TDA coming from the above described chemical-enzymatic-enzymatic process without crystallization. Losses of the amino β-lactam nucleus during purification and/or isolation steps are thus avoided, and investment in purification equipment is thus minimized, as the purification equipment formerly used for isolation of TDA can now be used for isolation of cefazolin.

It is an important feature of this invention that it is possible to achieve a high yield of β-lactam antibiotic in a reasonable reaction time (less than 3 hours) by supersaturating with the β-lactam nucleus and the acylating agent, but it is specially surprising that this high yield is obtained when the acylating agent is added as an crude mixture with some un-reacted acid obtained by a simple, cheap and economical chemical process. In this respect, it should be noted that there is not only a remarkable reduction of cost in what regards the reagents, but also no by-products are formed, increasing the final overall yield.

Advantageously, the high conversion of TDA obtained according to this invention (above 97%) avoids the need of reusing the remaining TDA after the enzymatic process, since at such a low percentage of TDA (less than 3%), it does not co-crystallize with cefazolin when the pH is shifted to pH 1.5. This results in a white cefazolin precipitate without TDA with a high purity of about 99.0-100.0 %. This does not only represent a reduction of the downstream process, but also less color, with the concomitant increase in the quality of the product. In a further aspect, the process is quite gentle to provide a final compound I without the presence of any heterocyclic thiol, for example MMTD, coming from the decomposition of the compound II, usually found in other known processes.

In an additional aspect, this invention relates to a process wherein the pH gradients inside the enzyme carrier are controlled by producing a buffer as a co-adjuvant to solubilize compound II. This aspect of the invention extents the half life of the enzyme and reduces the mass-transfer limitations between the substrates and/or products which occurs during the enzymatic process due to the inherent carrier properties.

In another advantageous aspect, the process includes the step of not controlling the pH after the conversion of the compound I is over 92 % of the original β-lactam nucleus, to achieve the maximal conversion. In still a further aspect, the process can achieve this maximal conversion by decreasing the temperature below 4°C or by a combination of both pH and temperature.

In an additional aspect, the process is preferably carried out in a batch mode with different sources of PGA immobilized on several suitable solid supports, cross-linked crystals or aggregates. Continuous or column processes are also possible but more expensive in term of equipment to finely control the critical pH changes in this process.

The advantages of this invention are, *inter alia,* as follows:
1. the high conversion of compound I obtained (above 97 %)
2. the high molar yield obtained after crystallization of compound I (above 88%)
3. the high reduction of cost by the use of an crude activated acylating agent, obtained by a simple and cheap chemical reaction.
4. the high solubility of compound II obtained which increases the yield in the crystallization process of the compound I at the end of the enzymatic synthesis.
5. the reduction of the equipment and the losses of the β-lactam nucleus by the use of solutions or moist cakes of compound II without any further purification or drying steps.
6. the increase of the number of reuses of immobilized enzyme by the use of a buffer solution in the process of solubilization of compound II.
7. the lack of by-products in the final reaction mixture.
8. the reduction of cost in terms of purification of compound I, since no compound II precipitates when the pH is decreased into the acidic range.
9. the lack of any halogenated and non-halogenated organic solvents or any dimethylaniline found in other chemical processes.
10. the resulting β-lactam antibiotic has a high purity not yet seen on the commercial bulk market without additional purifications steps.

Further, the present invention relates to a product of the above formula I, obtainable by the process according to the invention.

### Detailed description

The process according to the present invention uses, as starting material, a 3-position thiomethyl derivative of 7-ACA, the TDA [compound (II)] to produce cefazolin [compound (I)] with a high yield and selectivity, due to the presence of a penicillin G amidase (EC 3.5.1.11) and an activated acyl donor derived from 1H-tetrazol-1-acetic acid [compound (III)], where R is an alkyl, polyethylenglycol, ethylenglycol, amino group or secondary amides. The process can be illustrated in the following reaction scheme 1

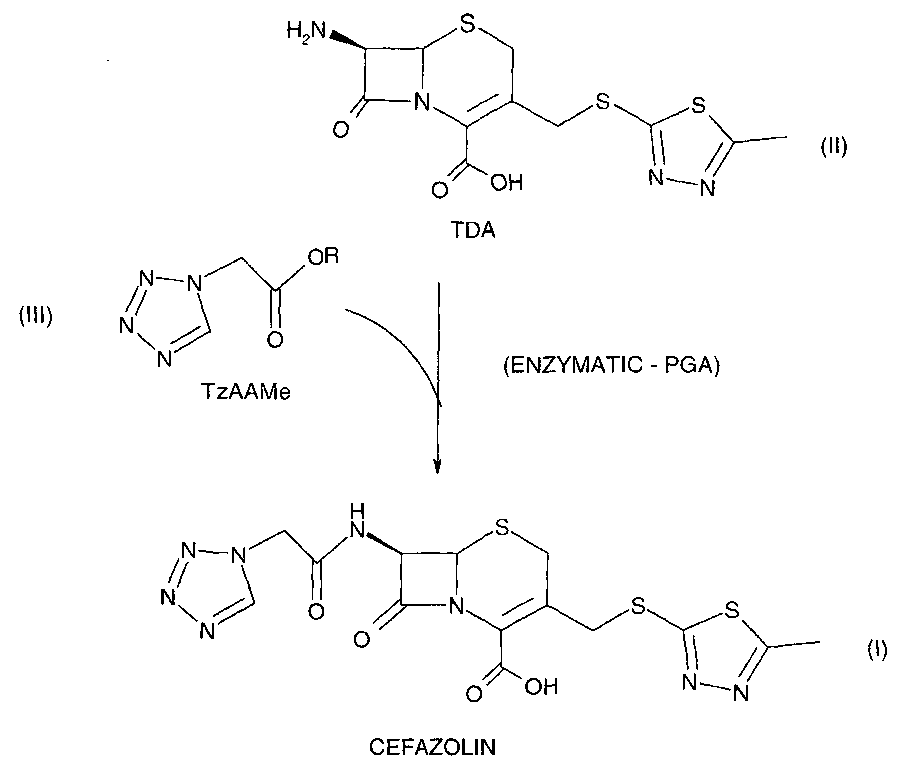

The acylating agent III is preferably selected from 1H-tetrazole-1-acetic acid or any suitable amide or any suitable ester thereof, 1H-tetrazole-1-acetic acid alkyl ester, hydroxiethyl ester or polyetilenglicol ester and 1H-tetrazole-1-acetic acid methyl ester, in crude or impure form.

According to a preferred embodiment, before the precipitation of the β-lactam antibiotic the biocatalyst is separated and recirculated to the process.

The penicillin amidase may be immobilized using a suitable cross-linker agent in a suitable solid support, in the form of crystal or aggregates of a size suitable for use as a biocatalyst, or in free soluble form, native or chemically and/ or genetically modified. The number of penicillin amidase units is preferably in the range form 100 to 50000 units per litre.

The enzymatic synthesis using a PGA may be carried out either as a kinetically-controlled reaction or as an equilibrium-controlled process. In the latter process, the enzyme only accelerates the rate at which the thermodynamic equilibrium is established. The direct condensation of the amino β-lactam with the corresponding free acid is carried out. The yields are determined by the thermodynamic equilibrium, and only the factors affecting it (pH, temperature, ionic, strength or solvent composition) can increase the amount of product. The reactions are slow, since low pH values have to be applied, values at which PGA is not so active and the use of organic co-solvents is sometime needed to change the pKa of the carboxylic acids, with the concomitant deleterious effect on PGA. This thermodynamically-controlled approach cannot be used in this invention since tetrazolyl acetic acid acts a strong acid. Therefore, it is dissociated at any pH.

In the kinetically-controlled approach, it is necessary to use an activated acyl donor (as an ester or amide) and to establish the optimal reaction time for condensation since the formation of the β-lactam product reaches a maximum, which is in general higher than in the thermodynamic equilibrium approach.

This maximum is due to a balance between three competitive reactions catalyzed by PGA in the reaction media: the synthesis of the antibiotic (transferase activity), the hydrolysis of the activated acyl donor (hydrolase activity) and the hydrolysis of the antibiotic that has been previously synthesized (amidase activity). These reactions occur in the cefazolin synthesis and are shown in the following scheme:

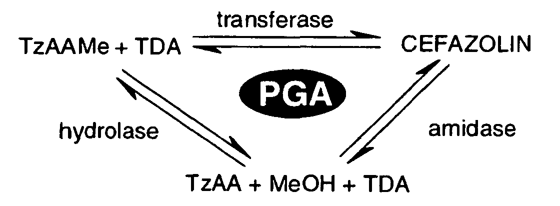

The parameters that control the maximum yield are: the degree of saturation of the active center of the enzyme by the acyl donor, the ratio transferase/hydrolase and the ratio transferase/amidase. Increasing the latter ratio improves the yield of the desired β-lactam product, while the ratio transferase/hydrolase determines the maximum theoretical yield in the absence of amidase activity. The variety of experimental conditions to be tuned in this kinetic approach are part of the embodiment of the invention.

The conditions applied in a method according to the invention depend on various parameters, in particular the type of reagents, the concentration of the reagents, reaction time, titrant, temperature, pH, enzyme concentration, enzyme source, enzyme support and the presence of additives in the reaction medium.

The optimum reaction temperature in a method according to the invention lies between -20 °C and 40 °C, preferably between 4 and 10 °C. The optimum pH in the preparation of a β-lactam antibiotic according to the invention lies between 6.0 and 8.0. In this regard, it is to be noted that it is highly preferable to perform a method according to the invention at pH 7.0 to 7.5, where the 3-modified amino-β-lactam has its maximum solubility in aqueous solution. The use of organic solvents, which would lead to effluent problems and shorten the life of the enzyme, is thus circumvented by the use of a base, preferably an inorganic base, in particular ammonia, potassium hydroxide or sodium hydroxide, but preferably ammonia.

Generally, the reagents are present at a higher concentration compatible with their solubility, specially in the case of 3-modified 7-amino β-lactam, TDA, below 120 mM, but preferably about 65 mM, depending on the pH of the reaction. To achieve this, an inorganic base, preferably ammonia 0.3 M, is added to an organic or inorganic acid, whose pKa is close to those used in the enzymatic reaction, to produce a buffer with a pH of about 6.0, prefereably between 6.5 and 8.7, in a concentration below 0.3 M, preferably below 0.15 M. Alternatively, the same buffer can be prepared by using a suitable non-toxic salt of the said inorganic acid, preferably an ammonium salt. Preferably, the buffer is phosphate buffer, more preferably ammonium phosphate buffer. To this solution TDA is added, and the pH is increased again with the same base solution to a pH in the range of 8.0 to 9.0, preferably about 8.6-8.7. At such pH, the TDA solution became clear, and it is then decreased back to the optimal enzymatic pH for the synthesis of compound I, by using an inorganic acid, such as hydrochloric acid, sulfuric acid, nitric acid, and preferably phosphoric acid.

In addition, the buffer thus formed became a preferred embodiment of the invention since it minimizes the pH-shift inside the enzyme carrier, thus increasing the half life of the enzyme and avoids mass-transfer limitations.

The precursor for the 7-position side chain of the β-lactam antibiotic to be prepared by a method according to the invention may be any compound that is recognized by PGA and leads to the desired cefazolin. Preferably, the substrate is an activated form of 1H-tetrazol-1-yl-2-acetic acid, such as an amide or alkyl ester, preferable a methyl ester, produced crude (about 92% purity) by boiling under reflux the said acid suspended in methanol in the presence of sulfuric acid for 3 hours, cooling down to room temperature, neutralizing the solution with a base (sodium bicarbonate or sodium hydroxide), filtering and drying under vacuum. Pure solution of the said methyl ester can be obtained after a time-consuming purification procedure known in the art, or by using diazomethane, but the latter substance is teratogenic. Other esters such as glycerin ester, polyethylene glycol ester monomethyl ether or hydroxyethyl ester can be obtained by known methods in the art, but with more complicated and expensive synthetic procedures compared with the first one described.

In a preferred embodiment of the process, this methyl activated acylating agent is used crude (about 92 % purity) in the range of 1.2 to 10 molar excess compared to the amino β-lactam nucleus, preferably about 8 molar excess.

A suitable penicillin G amidase (EC 3.5.1.11) can be used in a method according to the invention. The enzyme can be of plant, animal, fungal or bacterial origin or obtained by recombinant methods. The bacterial or fungal amidases are especially important as far as production, efficiency cost and stability are concerned. Representative bacteria or fungi for the amidase source include strains of microorganisms belonging to, for example, genera *Acetobacter, Achromobacter, Aeromonas, Alcaligenes, Aphanocladium Arthobacter, Brevibacterium, Beneckea, Bacillus, Cephalosporium, Corynebacterium, Escherichia, Flavobacterium, Gluconobacter, Kluyvera, Microbacterium, Micrococcus, Mycoplasma, Nocardia, Proteus, Pseudomonas, Providencia, Rhodopseudomonas, Spirilum, Staphylococcus or Xanthomonas,* or natural or artificial mutants or variants of them capable of producing amidase for the reaction of this invention. An especially useful enzyme is penicillin G amidase which can be obtained from *Escherichia coli* by recombinant methods, which are well known in the art.

The enzyme can be used in free form but preferably in an immobilized or crystallized form since the enzyme can be easily isolated and re-used. Different supports or immobilization technologies can be used, as described for this enzyme in EP 0 222 462, WO 92/12782 and EP 0 453 047. Commercially available immobilized penicillin G amidase can be obtained from Roche Molecular Biochemicals (Penzberg, DE) under the name "PGA-450", from Recordati (It) as described in EP 0 473 008, from DSM (Delft, NL) under the name "Assamblase®" and immobilized using gelatin and chitosan as gelating agent and glutaraldehyde as crosslinking agent. In addition, stable crystals of PGA are also available as CLEC enzymes from Altus (Boston, MA, USA) under the name "synthaCLEC ".

The amount of enzyme is chosen independently of the commercial origin and form such that the total reaction time does not exceed 3 hours. For the conversion of about 65 mM of substrate (TDA) into product in about two hours, 200 to 50000 enzyme reaction units per liter should be used, preferably about 5000 U/L. One unit of penicillin amidase corresponds to the amount of enzyme which hydrolyzes 1µmol of penicillin G per minute at pH 7.6 and 37°C.

The process of the invention is highly preferred to be carried out as a batch process, which means that the complete process is taking place in one reactor vessel. In other words, essentially no major reaction components are drawn off out of the reaction vessel at any time during the time of the reaction. If desired, the reaction can also be carried out continuously with in-line control of the concentration of the dissolved β-lactam nucleus. A percolation column design containing the immobilized or crystallized enzyme is also possible, but a bioreactor is desired since pH is more easily controlled by an autotitrator than in a column design and also a nitrogen flow of 0.01 vol/vol/min can be passed through a bottom diffuser of the bioreactor to increase the half-life of the enzyme under industrial scale.

Optionally, some additives can be applied in the method according to the invention to distort the enzyme microenvironment in order to improve the synthesis of desired β-lactam antibiotic. Among them, ethylenglycol, water activity depressors, such as polyethylenglycol (PEG), or penicillin G amidase inhibitors, such as phenylacetic acid and 2-thiophen acetic acid, can be used to minimize the hydrolysis of the desired product. But surprisingly, these additives are not needed in the process of this invention, since the high excess of tetrazolyl acetic acid in the reaction medium acts as an inhibitor of the hydrolysis of cefazolin. Further, the acylation reaction may be performed in the presence of nitrogen.

In a preferred embodiment of the invention, the reaction is optimized to attaining the maximum conversion of the desired β-lactam antibiotic by not controlling the pH after the conversion is above 92%. Another suitable embodiment on this respect is to lower the temperature of the reaction medium below 4°C. A combination of both embodiments is also possible.

In a preferred embodiment of the invention, a process to avoid the main problem of β-lactam enzymatic production processes is developed for the recovery of the desired product from complex reaction mixture. In many cases, downstream processing is hampered by losses of product resulting from degradation and mother liquor disposal. In this invention, once the reaction has been stopped at the maximum conversion, the reaction mixture is filtered on the molecular sieve of the bioreactor while stirring and the immobilized enzyme is reused in the interest of the process economics. Subsequently, valuable components such as the antibiotic and TzAA can be recovered in solution by the process according to the invention.

Solid antibiotic is then precipitated by a pH-shift. In the process of the invention, the pH can be decreased in several ways, for instance by adding an acid to the mixture. Suitable acids are for example inorganic acids, in particular sulfuric acid, hydrochloric acid or nitric acid. Preferably, hydrochloric acid is used.

In a preferred embodiment of the process, the pH is decreased below pH 3.5 without any precipitation of TDA in the acidic range of the pH, which are would not only hamper the downstream processing, but also tan the cefazolin crystals. The excess TzAA remaining in the mother liquor after cefazolin precipitation is reused by known methods, such as those described in GB 1 435 809, which are included in the present invention by reference.

As indicated above, the present invention further is related to a product according to the above formula I, obtainable by the process according to the invention. Said product is characterized in that it has a purity above 99.0 % by HPLC without any further purification, it contains less than 0.2 % of compound II, it contains less than 0.1 % of any heterocyclic thiol coming from the degradation of compound II, it is free of halogenated organic solvents, of non-halogenated organic solvents and of dimethylanilide.

The following examples are meant to illustrate the invention without limitation as to its generality.

### Examples

### Example 1

### Enzymatic synthesis of cefazolin free acid with crude tetrazolyl acetic acid methyl ester and penicillin G amidase immobilized on oxirane carrier

### STEP A: Preparation of tetrazolyl acetic acid methyl ester solution

0.1 mol of (1*H*-tetrazol-1-yl)-2-acetic acid, 1 mol of dry methanol and 0.3 mL of concentrated sulfuric acid were refluxed for 3 hours until the suspension converted to a clear solution. After cooling to room temperature, 0.01 mol of sodium bicarbonate was added to neutralize the solution and the residue was removed by filtration. The solution was then concentrated under vacuum at 40°C to remove the methanol excess, obtaining the ester as an oily material which turned solid after cooling below 25°C. The solid has a 92% of tetrazolyl acetic acid methyl ester by HPLC.

8.1 g of the impure (92%) tetrazolyl acetic acid methyl ester (52.4 mmol) obtained by the above process was suspended in 44 mL water at 4 °C under vigorous stirring in 60 minutes to obtain a clear solution. During this process the temperature was maintained below 25 °C

### STEP B: Enzymatic reaction in presence of immobilized penicillin G amidase

An enzymatic reactor (125 mL) was charged with 4.58 g of penicillin G amidase from *E. coli* immobilized on macroporus beads containing oxirane groups corresponding to 500 U, and obtained by separating the immobilized enzyme from an enzyme slurry, washing the beads with water for 1 hour at pH 7.5 and 4 °C, and finally weighing the beads without drying. In a second vessel (100 mL), 1.15 ammonium phosphate monobasic (10.0 mmol) was dissolved in 32 mL water and the pH was adjusted to about 6.0 with 3M ammonia. To this solution, 2.2 g (6.3 mmol) TDA was added and under vigorous stirring the pH was adjusted to pH 8.6 with 3M ammonia for a few minutes before decreasing back the pH to 7.5 with phosphoric acid at a temperature below 25 °C. Then the solution was transferred to the enzyme reactor. At 4°C, the reactor was supplied with the TzAAMe solution prepared in STEP A, and pH was maintained at pH 7.5 by means of titrating with 3 M ammonia. The kinetic of the process was followed by HPLC on a reverse phase column (Zorbax C8 5 µm l50x4.6 mm); the mobile phase was 10 mM tetrabutylammonium hydrogen sulphate, 15 mM potassium dihydrogen phosphate, pH 6.5 containing 30 % methanol at 1 mL/min with a 270 and 215 nm detection. The TzAAMe appeared at 2.6 minutes, the TZAA appeared at 2.9 minutes, the TDA appeared at 7.1 minutes and the Cefazolin at 10.6 minutes.

Representative samples of the reaction mixture were taken and when 92 % of conversion of TDA was achieved, the pH was not controlled to maximize the yield of cefazolin, unless it decreased below about pH 7.0. After 105 minutes, the enzyme reactor contained 97.4 % cefazolin (270 nm), 2.6 % TDA (270 nm), 1.6 % TzAAMe (215 nm) and 98.4 % TzAA (215 nm).

### STEP C: Isolation of cefazolin free acid

The slurry of cefazolin as obtained in STEP B was filtered off in combination with slow stirring. The remaining enzyme was washed with 6 mL water at 10 °C, being ready for a new cycle. The latter volume was added to the filtrate containing cefazolin and cooled down to 4°C. The pH was then adjusted to pH 1.5 with 3 M hydrochloric acid and allowed to crystallize for 2 hours under slow stirring.

The resulting white slurry was filtered off, washed at 4°C with 2 x 20 mL water at pH 1.5 and dried at 37 °C under vacuum to obtain 2.62 g (K.F. 0.9%) of substantially pure cefazolin, having 20 µL of a solution of 1 mg/mL an HPLC purity of 99.5 %. The overall molar yield from the TDA to cefazolin was 88.2 %.

### Example 2

### Enzymatic synthesis of cefazolin free acid with crude tetrazolyl acetic acid methyl ester and soluble penicillin G amidase

The reaction conditions were the same as in Example 1 but using 5kU/L of soluble penicillin G amidase *from E. coli* without immobilization in any support.

Representative samples of the reaction mixture were taken and when 92 % of conversion of TDA was achieved, the pH was not controlled to maximize the yield of cefazolin, unless it decreased below about pH 7.0. After 105 minutes, the enzyme reactor contained 98.5 % cefazolin (270 nm), 1.5 % TDA (270 nm), 0.9 % TzAAMe (215 nm) and 99.1 % TzAA (215 nm).

### Comparative Example 1

### Enzymatic synthesis of cefazolin free acid with pure tetrazolyl acetic acid methyl ester and penicillin G amidase immobilized on oxirane carrier

The reaction mixture were the same as in as in Example 1 but using 7.52 g of pure TzAAMe (99.1 % by HPLC)

Representative samples of the reaction mixture were taken and when 92 % of conversion of TDA was achieved, the pH was not controlled to maximize the yield of cefazolin, unless it decreased below about pH 7.0. After 105 minutes, the enzyme reactor contained 95.1 % cefazolin (270 nm), 4.9 % TDA (270 nm), 2.09 % TzAAMe (215 nm) and 97.9 % TzAA (215 nm).

### Comparative Example 2

### Enzymatic synthesis of cefazolin free acid with crude tetrazolyl acetic acid methyl ester and penicillin G amidase immobilized from Roche (PGA-450)

The reaction conditions were the same as in Example 1 but using 3.16 g of wet penicillin G amidase (PGA-450) from Roche Molecular Biochemicals (Penzberg, DE) corresponding to 5kU/L.
Representative samples of the reaction mixture were taken and when 92 % of conversion of TDA was achieved, the pH was not controlled to maximize the yield of cefazolin, unless it decreased below about pH 7.0. After 105 minutes, the enzyme reactor contained 92.0 % cefazolin (270 nm), 8.0 % TDA (270 nm), 0.44 % TzAAMe (215 nm) and 99.56 % TzAA (215 nm).

## Claims

1. A process for preparing the 7-(1-H-tetrazol-1-yl) acetamide-3-(2-methyl-1,3,4-thiadiazol-5-yl)thiomethyl-3-cephem-4-carboxylic acid or cefazolin of the formula: 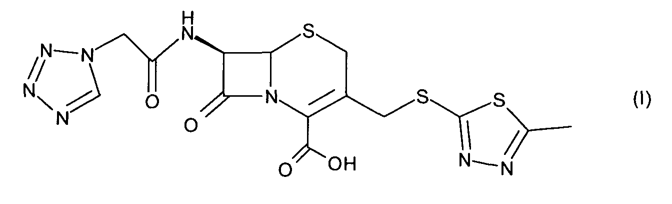 comprising catalysing the acylation of the amino β-lactam, the 7-amino-3-[5methyl-1,3,4-thiadiazol-2-yl)thiomethyl]-3-cephem-4-carboxylic acid, of the formula: 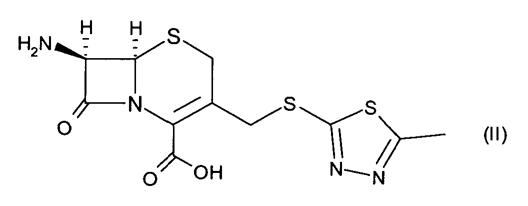 with an activated acylating agent derived from acetic acid of the formula: 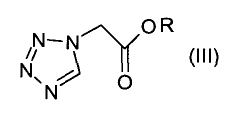 wherein R is an ester or amide of said acid, in the presence of a biocatalyst comprising penicillin amidase (EC 3.5.1.11), at a pH between 6.0 and 8.0, said pH being controlled bv means of a buffer comprising an inorganic base, and at a temperature between -20 °C and 40 °C, precipitating the desired β-lactam antibiotic by adding an acid to lower the pH below 3.5 at temperature in the range of 0°C to +20°C without the precipitation of remaining compound II in the acidic range of pH.

2. A process according to claim 1, wherein before the precipitation of the β-lactam antibiotic the biocatalyst is separated and recirculated to the process.

3. A process according to anyone of claims 1 and 2, wherein the acylation is performed at a temperature in the range from about 0°C to +10°C.

4. A process according to anyone of claims 1 to 3, wherein the solubility of compound II is regulated by using a combination of an inorganic base and an inorganic acid or a non-toxic salt of the said inorganic acid to produce a buffer solution.

5. A process according to claim 4, wherein the pH is regulated between 6.5 and 8.7.

6. A process according to anyone of claims 4 and 5, wherein the buffer is phosphate buffer.

7. A process according to anyone of claims 4 and 5, wherein the buffer is ammonium phosphate buffer.

8. A process according to anyone of claims 4 to 9, wherein the buffer concentration is below 0.3 M.

9. A process according to anyone of claims 1 to 8, wherein the acylating agent is 1 H-tetrazole-1-acetic acid, or any suitable amide or any suitable ester thereof.

10. A process according to anyone of claims 1 to 10, in which the acylating agent is 1H-tetrazole-1-acetic acid alkyl ester, hydroxyethyl ester or polyethylenglycol ester.

11. A process according to anyone of claims 1 to 10, wherein the acylating agent is 1 H-tetrazole-1-acetic acid methyl ester.

12. A process according to anyone of claims 1 to 11, in which the activated acylating agent is crude or impure.

13. A process according to anyone of claims 1 to 12, wherein the initial concentration of the acylating agent in the reaction mixture is between 1.2 and 10 molar excess in relation to the amino-β-lactam.

14. A process according to anyone of claims 1 to 13, wherein the initial concentration of the acylating agent in the reaction mixture is about 8 molar excess in relation to the amino-β-lactam.

15. A process according to anyone of claims 1 to 14, wherein the amino-β-lactam nucleus is 7-amino-3-[(5-methyl-1,3,4-thiadiazol-2-yl)thiomethyl]-3-cephem-4-carboxilic acid or a non toxic salt thereof.

16. A process according to anyone of claims 1 to 15, wherein the compound II is solubilized prior to the acylation by increasing the pH above pH 8.0 in an inorganic buffer and decreasing back to a neutral pH by using an inorganic acid.

17. A process according to anyone of claims 1 to 16, wherein the penicillin G amidase (EC 3.5.1.11) is obtained from an *Acetobacter, Achromobacter, Aeromonas, Alcaligenes, Aphanocladium Arthobacter, Brevibacterium, Beneckea, Bacillus, Cephalosporium, Corynebacterium, Escherichia, Flavobacterium,* *Gluconobacter, Kluyvera, Microbacterium, Micrococcus, Mycoplasma, Nocardia, Proteus, Pseudomonas, Providencia, Rhodopseudomonas, Spirilum, Staphylococcus or Xanthomonas species,* or natural or artificial mutants or variants of them.

18. A process according to anyone of claims 1 to 17, wherein the penicillin amidase is immobilized using a suitable cross-linker agent in a suitable solid support.

19. A process according to anyone of claims 1 to 18, wherein the penicillin amidase is in the form of crystal or aggregates of a size suitable for use as a biocatalyst.

20. A process according to anyone of claims 1 to 18, wherein the penicillin amidase is in free soluble form, native or chemically and/or genetically modified.

21. A process according to anyone of claims 1 to 20, wherein the process is carried out while maintaining the penicillin amidase in dispersion in aqueous substrate mixture solution.

22. A process according to anyone of claims 1 to 21, wherein the reaction is performed with the number of penicillin amidase units is in the range from 100 to 50000 units per litre.

23. A process according to anyone of claims 1 to 22, wherein the process is carried out as a one-pbt batch process.

24. A process according to anyone of claims 1 to 22, wherein the process is carried out as continuous process.

25. A process according to anyone of claims 1 to 22, wherein the process is carried out in a column.

26. A process according to anyone of claims 1 to 25, wherein the process is carried out in the presence of additives to decrease the hydrolysis of the compound I.

27. A process according to claim 26, wherein the additives are inhibitors of penicillin G amidase or distort its microenvironment.

28. A process according to claim 1 to 27, wherein the acylation is performed in the presence of nitrogen.

29. A process according to anyone of claims 1 to 28, wherein the pH is maintained constant during the time of the reaction between pH 7.3 and 8.2.

30. A process according to anyone of claims 1 to 29, wherein the pH is maintained constant during the time of the reaction, preferably at pH about 7.5.

31. A process according to anyone of claims 1 to 29, wherein the pH is not controlled after the synthesis of the compound I is above 92%, to achieve maximal conversion.

32. A process according to anyone of claims 1 to 31, wherein the temperature is maintained constant during the time of the reaction between -10°C and 15°C.

33. A process according to anyone of claims 1 to 32, wherein the temperature is maintained constant during the time of the reaction, preferably at about 4°C.

34. A process according to anyone of claims 1 to 33, wherein the temperature is decreased after the synthesis of the compound I is above 92%, to achieve maximal conversion.

35. A process according to anyone of claims 1 to 29 and 34, wherein' both the temperature and pH are decreased after the synthesis of the compound I is above 92%, to achieve maximal conversion.

36. A process according to anyone of claims 1 to 35, wherein the pH of the solution containing compound I is decreased below 3.5 with a suitable organic or inorganic acid to precipitate it.

37. A process according to anyone of the claims 1 to 36, wherein the antibiotic of formula I is cefazolin.

## Patentansprüche

1. Verfahren für die Zubereitung der 7-(1-H-Tetrazol-1-yl)acetamid-3-(2-methyl-1,3,4-thiadiazol-5-yl)thiomethyl-3-cephem-4-carbonsäure oder Cefazolin der Formel: 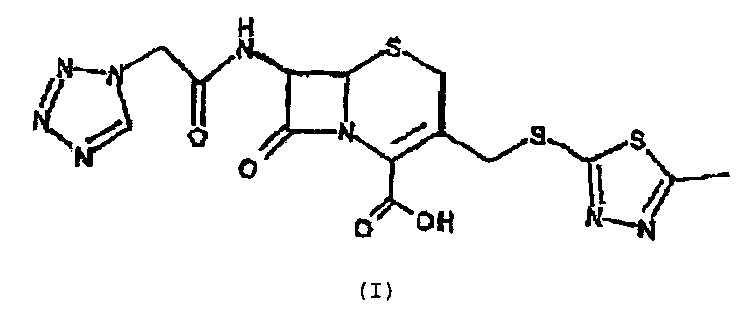 umfassend das Katalysieren der Acylierung des Amino-β-lactams, der 7-Amino-3-[5-methyl-1,3,4-thiadiazol-2-yl)thiomethyl]-3-cephem-4-carbonsäure, der Formel: 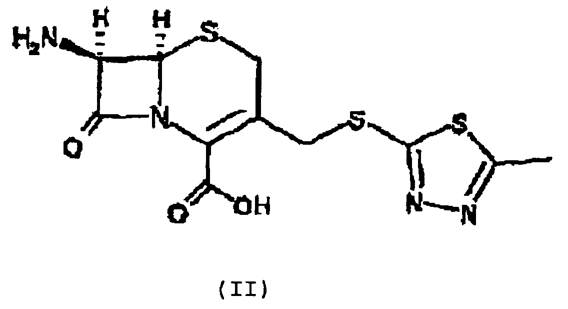 mit einem aktivierten Acylierungsmittel deriviert von Essigsäure der Formel: 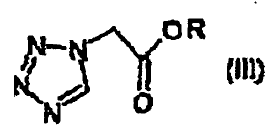 wobei R ein Ester oder Amid der Säure ist, in Anwesenheit eines Biokatalysators umfassend Penicillinamidase (EC 3.5.1.11), bei einem pH-Wert zwischen 6,0 und 8,0, wobei der pH-Wert durch einen Puffer eingestellt wird umfassend eine anorganische Base, bei einer Temperatur zwischen - 20 °C und 40 °C, das Ausfällen des erwünschten β-Lactam-Antibiotikums durch Zusetzen einer Säure zum Senken des pH-Werts unter 3,5 bei einer Temperatur im Bereich von 0 °C bis + 20 °C ohne Ausfällen der verbleibenden Verbindung II im sauren pH-Bereich.

2. Verfahren nach Anspruch 1, wobei vor dem Ausfällen des β-Lactam-Antibiotikums der Biokatalysator abgetrennt und in den Prozess rückgeführt wird.

3. Verfahren nach einem der Ansprüche 1 und 2, wobei die Acylierung bei einer Temperatur im Bereich von 0 °C bis + 10 °C durchgeführt wird.

4. Verfahren nach einem der Ansprüche 1 und 3, wobei die Löslichkeit der Verbindung II durch Verwendung einer Kombination einer anorganischen Base und einer anorganischen Säure oder eines nichttoxischen Salzes der anorganischen Säure unter Bildung einer Pufferlösung reguliert wird.

5. Verfahren nach Anspruch 4, wobei der pH-Wert auf zwischen 6,5 und 8,7 reguliert wird.

6. Verfahren nach einem der Ansprüche 4 und 5, wobei der Puffer Phosphatpuffer ist.

7. Verfahren nach einem der Ansprüche 4 und 5, wobei der Puffer Ammoniumphosphatpuffer ist.

8. Verfahren nach einem der Ansprüche 4 bis 9, wobei die Pufferkonzentration unter 0,3 M liegt.

9. Verfahren nach einem der Ansprüche 1 bis 8, wobei das Acylierungsmittel 1H-Tetrazol-1-essigsäure oder irgendein geeignetes Amid oder irgendein geeigneter Ester desselben ist.

10. Verfahren nach einem der Ansprüche 1 bis 10, wobei das Acylierungsmittel 1H-Tetrazol-1-essigsäurealkylester, Hydroxyethylester oder Polyethylenglykolester ist.

11. Verfahren nach einem der Ansprüche 1 bis 10, wobei das Acylierungsmittel 1H-Tetrazol-1-essigsäuremethylester ist.

12. Verfahren nach einem der Ansprüche 1 bis 11, wobei das aktivierte Acylierungsmittel roh oder unrein ist.

13. Verfahren nach einem der Ansprüche 1 bis 12, wobei die Anfangskonzentration des Acylierungsmittels in der Reaktionsmischung im molaren Überschuss von 1,2 bis 10 mit Bezug auf das Amino-β-lactam vorliegt.

14. Verfahren nach einem der Ansprüche 1 bis 13, wobei die Anfangskonzentration des Acylierungsmittels in der Reaktionsmischung im molaren Überschuss von ca. 8 mit Bezug auf das Amino-β-lactam vorliegt.

15. Verfahren nach einem der Ansprüche 1 bis 14, wobei der Amino-β-lactamkern in 7-Amino-3-[(5-methyl-1,3,4-thiadiazol-2-yl)thiomethyl]-3-cephem-4-carbonsäure oder ein nichttoxisches Salt derselben ist.

16. Verfahren nach einem der Ansprüche 1 bis 15, wobei die Verbindung II vor der Acylierung löslich gemacht wird durch Erhöhen des pH-Werts über pH 8,0 in einem anorganischen Puffer und Reduzieren desselben zurück auf einen neutralen pH-Wert durch Verwendung einer anorganischen Säure.

17. Verfahren nach einem der Ansprüche 1 bis 16, wobei die Penicillin G-Amidase (EC 3.5.1.11) von einer Actobacter-, Achromobacter-, Aeromonas-, Alcaligenes-, Aphanocladium, Arthobacter-, Brevibacterium-, Beneckea-, Bacillus-, Cephalosporium-, Corynebacterium-, Escherichia-, Flavobacterium-, Gluconobacterium-, Kluyvera-, Microbacterium-, Micrococcus-, Mycoplasma-, Nocardia-, Proteus-, Pseudomonas-, Providencia-, Rhodopseudomonas-, Spirilum-, Staphylococcus- oder Xanthomonas-Spezies oder natürlichen oder künstlichen Mutanten oder Varianten derselben erhalten wird.

18. Verfahren nach einem der Ansprüche 1 bis 17, wobei die Penicillinamidase mit Hilfe eines geeigneten Vernetzungsmittels in einem geeigneten festen Träger immobilisiert wird.

19. Verfahren nach einem der Ansprüche 1 bis 18, wobei die Penicillinamidase in Form eines Kristalls oder von Aggregaten einer zur Verwendung als Biokatalysator geeigneten Größe vorliegt.

20. Verfahren nach einem der Ansprüche 1 bis 18, wobei die Penicillinamidase in frei löslicher Form, nativ oder chemisch und/oder genetisch modifiziert ist.

21. Verfahren nach einem der Ansprüche 1 bis 20, wobei das Verfahren durchgeführt wird, während die Penicillinamidase in Dispersion in wässriger Substratmischungslösung gehalten wird.

22. Verfahren nach einem der Ansprüche 1 bis 21, wobei die Reaktion durchgeführt wird, während die Anzahl Penicillinamidaseeinheiten im Bereich von 100 bis 50,000 Einheiten pro Liter liegt.

23. Verfahren nach einem der Ansprüche 1 bis 22, wobei das Verfahren als Eintopfchargenverfahren durchgeführt wird.

24. Verfahren nach einem der Ansprüche 1 bis 22, wobei das Verfahren als kontinuierliches Verfahren durchgeführt wird.

25. Verfahren nach einem der Ansprüche 1 bis 22, wobei das Verfahren in einer Säule durchgeführt wird.

26. Verfahren nach einem der Ansprüche 1 bis 25, wobei das Verfahren in Gegenwart von Zusatzmitteln zum Reduzieren der Hydrolyse der Verbindung I durchgeführt wird.

27. Verfahren nach Anspruch 26, wobei die Zusatzmittel Inhibitoren der Penicillin G-Amidase sind oder ihr Mikroumfeld verzerren,

28. Verfahren nach einem der Ansprüche 1 bis 27, wobei die Acylierung in Gegenwart von Stickstoff durchgeführt wird.

29. Verfahren nach einem der Ansprüche 1 bis 28, wobei der pH-Wert während der Zeit der Reaktion zwischen pH 7,3 und 8,2 konstant gehalten wird.

30. Verfahren nach einem der Ansprüche 1 bis 29, wobei der pH-Wert während der Zeit der Reaktion konstant, bevorzugt bei einem pH-Wert von 7,5 gehalten wird.

31. Verfahren nach einem der Ansprüche 1 bis 29, wobei der pH-Wert nachdem die Synthese der Verbindung I bei über 92 % liegt, nicht eingestellt wird, um eine maximale Umwandlung zu erreichen.

32. Verfahren nach einem der Ansprüche 1 bis 31, wobei die Temperatur während der Zeit der Reaktion zwischen - 10 °C und 15 °C konstant gehalten wird.

33. Verfahren nach einem der Ansprüche 1 bis 32, wobei die Temperatur während der Zeit der Reaktion konstant, bevorzugt bei ca. 4 °C, gehalten wird.

34. Verfahren nach einem der Ansprüche 1 bis 33, wobei die Temperatur nachdem die Synthese der Verbindung I bei über 92 % liegt, reduziert wird, um eine maximale Umwandlung zu erreichen.

35. Verfahren nach einem der Ansprüche 1 bis 29, und 34 wobei sowohl die Temperatur als auch der pH-Wert, nachdem die Synthese der Verbindung I bei über 92 % liegt, reduziert werden, um eine maximale Umwandlung zu erzielen

36. Verfahren nach einem der Ansprüche 1 bis 35, wobei der pH-Wert der die Verbindung I enthaltenden Lösung mit einer geeigneten organischen oder anorganischen Säure zum Ausfällen derselben auf unter 3,5 reduziert wird.

37. Verfahren nach einem der Ansprüche 1 bis 36, wobei das Antibiotikum der Formel I Cefazolin ist.

## Revendications

1. Procédé de préparation de l'acide 7-(1-H-tétrazol-1-yl)acétamide-3-(2-méthyl-1,3,4-thiadiazol-5-yl)thiométhyl-3-céphème-4-carboxylique ou céfazoline de formule : 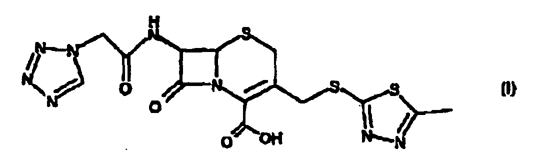 comprenant
la catalyse de l'acylation de l'amino-bêta-lactame, l'acide 7-amino-3-[(5-méthyl-1,3,4-thiadiazol-2-yl)thiométhyl]-3-céphème-4-carboxylique, de formule : 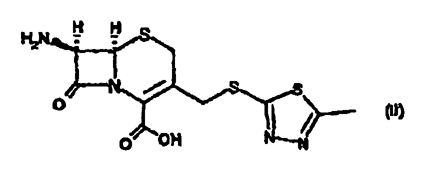 avec un agent d'acylation activé dérivé de l'acide acétique de formule : 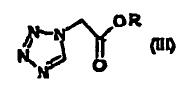 dans laquelle R représente un ester ou un amide dudit acide, en présence d'un biocatalyseur comprenant la pénicilline-amidase (EC 3.5.1.11), à un pH compris entre 6,0 et 8,0, ledit pH étant régulé à l'aide d'un tampon comprenant une base inorganique, et à une température comprise entre -20°C et 40°C,
la précipitation de l'antibiotique bêta-lactame souhaité au moyen de l'addition d'un acide pour abaisser le pH en dessous de 3,5, à une température située dans la gamme de 0°C à +20°C, sans la précipitation du composé II restant dans la gamme de pH acide.

2. Procédé selon la revendication 1, dans lequel, avant la précipitation de l'antibiotique bêta-lactame, le biocatalyseur est séparé et recyclé dans le procédé.

3. Procédé selon l'une quelconque des revendications 1 et 2, dans lequel l'acylation est réalisée à une température située dans la gamme d'environ 0°C à +10°C.

4. Procédé selon l'une quelconque des revendications 1 à 3, dans lequel la solubilité du composé II est régulée à l'aide d'une combinaison d'une base inorganique et d'un acide inorganique ou d'un sel non toxique dudit acide inorganique pour produire une solution tampon.

5. Procédé selon la revendication 4, dans lequel le pH est régulé entre 6,5 et 8,7.

6. Procédé selon l'une quelconque des revendications 4 et 5, dans lequel le tampon est un tampon phosphate.

7. Procédé selon l'une quelconque des revendications 4 et 5, dans lequel le tampon est un tampon phosphate d'ammonium.

8. Procédé selon l'une quelconque des revendications 4 à 9, dans lequel la concentration du tampon est inférieure à 0,3 M.

9. Procédé selon l'une quelconque des revendications 1 à 8, dans lequel l'agent d'acylation est l'acide 1H-tétrazol-1-acétique ou tout amide approprié ou tout ester approprié de celui-ci.

10. Procédé selon l'une quelconque des revendications 1 à 10, dans lequel l'agent d'acylation est l'ester alkylique, l'ester hydroxyéthylique ou l'ester polyéthylèneglycolique de l'acide 1H-tétrazol-1-acétique.

11. Procédé selon l'une quelconque des revendications 1 à 10, dans lequel l'agent d'acylation est l'ester méthylique de l'acide 1H-tétrazol-1-acétique.

12. Procédé selon l'une quelconque des revendications 1 à 11, dans lequel l'agent d'acylation activé est brut ou impur.

13. Procédé selon l'une quelconque des revendications 1 à 12, dans lequel la concentration initiale de l'agent d'acylation dans le mélange réactionnel est en excès molaire de 1,2 à 10 par rapport à l'amino-bêta-lactame.

14. Procédé selon l'une quelconque des revendications 1 à 13, dans lequel la concentration initiale de l'agent d'acylation dans le mélange réactionnel est en excès molaire d'environ 8 par rapport à l'amino-bêta-lactame.

15. Procédé selon l'une quelconque des revendications 1 à 14, dans lequel le noyau amino-bêta-lactame est l'acide 7-amino-3-[(5-méthyl-1,3,4-thiadiazol-2-yl)thiométhyl]-3-céphème-4-carboxylique ou un sel non toxique de celui-ci.

16. Procédé selon l'une quelconque des revendications 1 à 15, dans lequel le composé II est solubilisé avant l'acylation au moyen d'une élévation du pH au-dessus de pH 8,0 dans un tampon inorganique et de sa re-diminution jusqu'à un pH neutre à l'aide d'un acide inorganique.

17. Procédé selon l'une quelconque des revendications 1 à 16, dans lequel la pénicilline-G-amidase (EC 3.5.1.11) est obtenue à partir de l'une des espèces *Acétobacter, Achromobacter, Aeromonas, Alcaligenes, Aphanocladium Arthobacter, Brevibacterium, Beneckea, Bacillus, Cephalosporium, Corynebacterium, Escherichia, Flavobacterium, Gluconobacter, Kluyvera, Microbacterium, Micrococcus, Mycoplasma, Nocardia, Proteus, Pseudomonas, Providencia, Rhodopseudomonas, Spirilum, Staphylococcus* ou *Xanthomonas,* ou de variantes ou de mutants naturels ou artificiels de celles-ci.

18. Procédé selon l'une quelconque des revendications 1 à 17, dans lequel la pénicilline-amidase est immobilisée à l'aide d'un agent de réticulation approprié sur un support solide approprié.

19. Procédé selon l'une quelconque des revendications 1 à 18, dans lequel la pénicilline-amidase se présente sous la forme de cristaux ou d'agrégats dont la taille est appropriée à une utilisation en tant que biocatalyseur.

20. Procédé selon l'une quelconque des revendications 1 à 18, dans lequel la pénicilline-amidase se présente sous une forme soluble libre, naturelle ou chimiquement et/ou génétiquement modifiée.

21. Procédé selon l'une quelconque des revendications 1 à 20, dans lequel le procédé est réalisé tandis que la pénicilline-amidase est maintenue en dispersion dans une solution-substrat aqueuse mixte.

22. Procédé selon l'une quelconque des revendications 1 à 21, dans lequel la réaction est réalisée avec un nombre d'unités de pénicilline-amidase situé dans la gamme de 100 à 50 000 unités par litre.

23. Procédé selon l'une quelconque des revendications 1 à 22, dans lequel le procédé est réalisé sous forme de procédé discontinu en un seul récipient.

24. Procédé selon l'une quelconque des revendications 1 à 22, dans lequel le procédé est réalisé sous forme de procédé continu.

25. Procédé selon l'une quelconque des revendications 1 à 22, dans lequel le procédé est réalisé dans une colonne.

26. Procédé selon l'une quelconque des revendications 1 à 25, dans lequel le procédé est réalisé en présence d'additifs pour réduire l'hydrolyse du composé I.

27. Procédé selon la revendication 26, dans lequel les additifs sont des inhibiteurs de la pénicilline-G-amidase ou perturbent son micro-environnement.

28. Procédé selon les revendications 1 à 27, dans lequel l'acylation est réalisée en présence d'azote.

29. Procédé selon l'une quelconque des revendications 1 à 28, dans lequel le pH est maintenu à une valeur constante pendant la durée de la réaction, entre pH 7,3 et pH 8,2.

30. Procédé selon l'une quelconque des revendications 1 à 29, dans lequel le pH est maintenu à une valeur constante pendant la durée de la réaction, de préférence à environ pH 7,5.

31. Procédé selon l'une quelconque des revendications 1 à 29, dans lequel le pH n'est pas régulé après que la synthèse du composé I a dépassé 92%, pour permettre une conversion maximale.

32. Procédé selon l'une quelconque des revendications 1 à 31, dans lequel la température est maintenue à une valeur constante pendant la durée de la réaction, entre -10°C et 15°C.

33. Procédé selon l'une quelconque des revendications 1 à 32, dans lequel la température est maintenue à une valeur constante pendant la durée de la réaction, de préférence à environ 4°C.

34. Procédé selon l'une quelconque des revendications 1 à 33, dans lequel la température est abaissée après que la synthèse du composé I a dépassé 92%, pour permettre une conversion maximale.

35. Procédé selon l'une quelconque des revendications 1 à 29 et 34, dans lequel la température et le pH sont tous deux abaissés après que la synthèse du composé I a dépassé 92%, pour permettre une conversion maximale.

36. Procédé selon l'une quelconque des revendications 1 à 35, dans lequel le pH de la solution contenant le composé I est abaissé en dessous de 3,5 à l'aide d'un acide organique ou inorganique approprié pour provoquer sa précipitation.

37. Procédé selon l'une quelconque des revendications 1 à 36, dans lequel l'antibiotique de formule I est la céfazoline.
